# EUROPEAN PATENT APPLICATION

(11) **EP 4 520 428 A1**
(43) Date of publication of application: **12.03.2025**
(21) Application number: 23799252.4
(22) Date of filing: 04.05.2023
(51) Int. Cl.: B01J 19/00, B82Y 40/00, B01F 33/30

(54) **MICRO-FLUIDIC CHIP AND HIGH-THROUGHPUT NANO-PARTICLE SYNTHESIS SYSTEM BASED ON MICRO-FLUIDIC TECHNOLOGY**

(30) Priority: 05.05.2022 CN 202221044491 U
(71) Applicant: Suzhou Precigenome Ltd, Co;, Suzhou City, Jiangsu 215000 (CN)
(72) Inventor: WANG, Yaqi, Suzhou, Jiangsu 215000 (CN); XIA, Yiqiu, San Jose California 95131 (US); LING, Yunfeng, San Jose California 95131 (US); LIANG, Yuting, San Jose California 95131 (US); ZHANG, Hua, Suzhou, Jiangsu 215000 (CN)
(74) Representative: Lavoix
(86) International application number: PCT/CN2023/091999
(87) International publication number: WO 2023/213266

(57) **Abstract**

Provided in the present application are a microfluidic chip and a high-throughput small-volume nanoparticle synthesis system based on microfluidic technology. The system comprises a pressure controller and a pressure distribution assembly, which is connected to an output end of the pressure controller, wherein a pressure output end of the pressure distribution assembly is connected to a microfluidic chip, a flow channel for nano-particle synthesis is arranged on the microfluidic chip, and each of an inlet end and a synthesis end of the flow channel is provided with a liquid storage pool. The beneficial effects of the present application are embodied in that: the size, distribution and consistency of nano-particles can be accurately controlled, and reagent raw materials are directly synthesized by means of a flow channel, thereby avoiding a reagent waste caused by a dead volume due to a redundant connection, and a high-throughput requirement can be effectively met by means of increasing the number of microfluidic chips or the number of parallel flow channels on the same chip.

## Description

### TECHNICAL FIELD

The present application belongs to the field of microfluidic nanomaterial syntheis technology, and specifically relates to a microfluidic chip and a high-throughput nanoparticle synthesis system based on microfluidic technology.

### BACKGROUND

Nanoparticle synthesis technology is at the forefront of the rapidly developing field of nanotechnology. Its unique size-dependent characteristics give these materials great advantages in many fields and make them irreplaceable. This technology has been widely used in many industries, such as drug delivery, energy and electronics. Nanoparticle synthesis technology is one of the key steps to realize the application of nanoparticles.

Since the size characteristics of nanoparticles are critical in most applications, the size distribution, yield and size reproducibility between batches of nanoparticle synthesis are very important parameters in the evaluation of nanoparticle synthesis. One of the traditional methods for nanoparticle synthesis is based on the principle of batch mixing. Specifically, the raw materials for preparing nanoparticles are dissolved in an organic phase or an aqueous phase, and then added to another aqueous phase or an organic phase with poor compatibility therewith, and quickly mixed in a beaker or other equipment by stirring. However, when conventional batch synthesis methods (mixing in bulk solution) are used for large-scale production of nanoparticles, the quality of the synthesized particles is poor. There are also some uncontrollable factors, such as aggregation and heterogeneous mixing, which result in poor size uniformity and reproducibility of the nanoparticles.

Microreactors based on microfluidic technology can achieve rapid mixing of reagents, temperature control, and precise spatiotemporal manipulation of reactions. In nanoparticle synthesis using microfluidic technology, the mixing is controlled and uniform, thus nanoparticles of uniform size can be produced. At the same time, the reproducibility of the physicochemical properties of the nanoparticles can also be precisely controlled. In addition, by regulating the microenvironment of nanoparticle synthesis, the size uniformity and repeatability of nanoparticles can be further improved, thereby improving the yield of the nanoparticle preparation process.

Currently, the instruments on the market that use microfluidics to achieve nanoparticle synthesis mainly come from Canada's Precision Nanosystems and Precigenome. The instrument of Precision Nanosystems mainly uses a syringe pump to push the organic phase and aqueous phase into the microfluidic chip for mixing, and collects the synthesized nanoparticles at the outlet end of the microfluidic chip. This instrument achieves very high size controllability, uniformity and high reproducibility of nanoparticles. However, since it uses a syringe pump as the power to propel the fluid, it has some inherent shortcomings. For example, when the syringe pump adjusts the flow rate, it has some problems, such as large volume, slow response speed, low adjustment accuracy, pulsation of flow, low sample utilization efficiency, and easy contamination. The pulsation of flow and low adjustment accuracy will affect the uniformity of mixing during nanoparticle synthesis, thereby reducing the uniformity of nanoparticles. The syringe pump requires the reagent to be loaded into the syringe first, which is complicated to operate and prone to contamination. In addition, the capacity of the syringe is limited, and the instrument requires a lot of engineering design and improvement during the process of expanding production, which makes it inconvenient to use directly in high-throughput production. In addition, the use of syringe pumps also has insurmountable difficulties in achieving high throughput (multiple samples running simultaneously): when running multiple samples simultaneously, each sample needs to be configured with a separate syringe pump, which inevitably leads to system complexity and is very unfavorable for the expansion of the system in the direction of high throughput. At present, in order to achieve the production increase from small volume (less than 1ml) to large volume (greater than 1ml), these devices need to adopt microfluidic chips of different designs. Different designs of chips often also require different conditions for producing nanoparticles to produce similar nanoparticles. This not only increases the design and production costs, but also increases the technical difficulty of increasing mass production while maintaining the same performance.

Precigenome's instrument (patent application number 2021110609590) is an integrated device that can achieve fully automated nanoparticle synthesis based on pressure control and microfluidic mixing technology. This instrument partially makes up for the many shortcomings of the syringe pump used in Precision Nanosystems' instruments, and can realize a highly automated and continuous process for producing nanoparticles to increase the yield of nanoparticles. However, since the device uses many valves, flow meters and other fluid control components, these fluid control components and the pipes connecting them will bring a large dead volume, which is unable to meet the demand for high throughput and small volume required for in formulation development and optimization for nanoparticle synthesis. Therefore, developing a nanoparticle synthesis system that can achieve high throughput and small volume has great market application prospects.

### SUMMARY

In order to address the deficiencies of the prior art and to achieve high-throughput and small-volume nanoparticle synthesis, the present application provides a microfluidic chip and a high-throughput nanoparticle synthesis system based on microfluidic technology.

The purpose of this application is achieved through the following technical solutions:
A microfluidic chip based on microfluidic technology comprises a chip body, wherein at least one flow channel for synthesizing nanoparticles is provided on the chip body, wherein each of an inlet end andan synthesis end of the flow channel is provided with a liquid storage pool, and a central axis of the liquid storage pool is perpendicular to the flow channel.

Preferably, the liquid storage pool is integrally formed or detachably connected with the chip body. Preferably, detachable connection is formed between the liquid storage pool and the microfluidic chip through mutually matched Luer tapers.

Preferably, a high-throughput and small-volume nanoparticle synthesis system based on microfluidic technology comprises a pressure controller, and a pressure distribution assembly connected to an output end of the pressure controller, wherein a pressure output end of the pressure distribution assembly is connected to any one of the above microfluidic chips, and the number of the microfluidic chip is at least one.

Preferably, a sealing assembly is provided between the pressure distribution assembly and the microfluidic chip to form an airtight connection between the pressure distribution assembly and the microfluidic chip.

Preferably, the pressure distribution assembly comprises a pressure distribution plate, and the sealing assembly is a sealing gasket, which is provided with an opening and at least covers a top opening of the liquid storage pool to ensure the airtight connection between the pressure distribution assembly and the microfluidic chip.

Preferably, the sealing gasket is selectively connected to the pressure distribution plate or to a port of the liquid storage pool.

Preferably, a volume of the liquid storage pool is 20 µl-1 ml.

Preferably, the pressure controller is a multi-channel pressure controller, and the number of the channels is greater than or equal to 2.

Preferably, a control valve is provided between the pressure controller and the pressure distribution assembly.

The beneficial effects of the present application are as follows: the size, distribution and consistency of nanoparticles can be precisely controlled; reagent raw materials are directly for synthesis through the flow channel, thus avoiding waste of reagents caused by dead volume due to redundant connections; and high-throughput requirements can be well met by increasing the number of microfluidic chips or the number of parallel flow channels on the same chip.

### BRIEF DESCRIPTION OF THE DRAWINGS

FIG. 1: A schematic diagram of the principle structure of the synthesis system of the present application.
FIG. 2: A schematic diagram of the front cross-sectional structure of FIG. 1.
FIG. 3: A schematic diagram of an example structure of the present application.
FIG. 4: A schematic diagram of the structure of the air pressure distribution plate in FIG. 3 in the open state.
FIG. 5: Bar graph of the size and PDI of lipid nanoparticles synthesized by present system.
FIG. 6: A diagram of particle size and PDI of different batches of lipid nanoparticles synthesized using a low-throughput flow rate control device.
FIG. 7: Comparison chart of the particle size and size PDI of DNA-encapsulated lipid nanoparticles synthesized in different devices.
FIG. 8: Comparison chart of DNA encapsulation efficiency of lipid nanoparticles synthesized in different devices.
FIG. 9: Fluorescence and bright field images of HepG2 (top) and K562 (bottom) treated with synthetic GFP DNA-encapsulated lipid nanoparticles for 48 hours.

### DETAIL DESCRIPTION

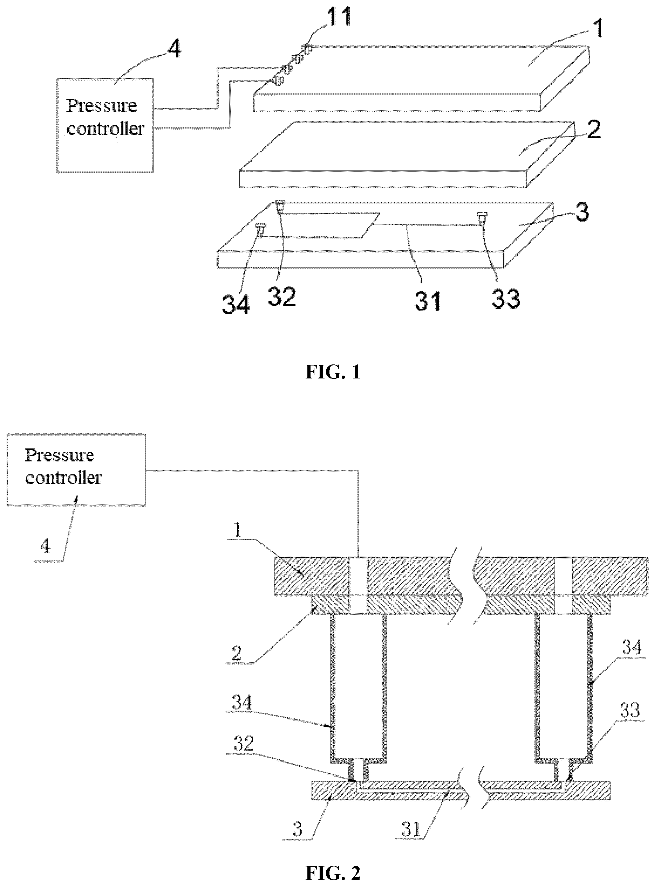

The present application discloses a microfluidic chip based on microfluidic technology, and a synthesis system for high-throughput and small-volume nanoparticle synthesis using the chip. As shown in FIGS. 1-2, the microfluidic chip 3 comprises a chip body, and a flow channel 31 for nanoparticle synthesis provided on the chip body, and a liquid storage pool 34 is provided at each of the inlet end 32 and the synthesis end 33 of the flow channel 31. The central axis of the liquid storage pool 34 is provided perpendicular to the flow channel. The liquid storage pool 34 and the microfluidic chip 3 are integrally injection-molded or detachably connected. The detachable connection means that the liquid storage pool 34 is connected with the microfluidic chip 3 through mutually matched Luer tapers. Of course, other connecting parts that match with each other may also be used. In the present application, the flow channel 31 is directly connected to the liquid storage pool 34, which can effectively avoid the connection of multiple flow valves or pipelines in the prior art and will not cause waste of reagents due to dead volume.

The present application also discloses a high-throughput nanoparticle synthesis system based on microfluidic technology using the microfluidic chip, comprising a pressure controller 4, and a pressure distribution assembly connected to the output end of the pressure controller 4, and the pressure output end of the pressure distribution assembly is connected to the microfluidic chip 3. The pressure distribution assembly comprises a pressure distribution plate 1, on which a pressure input end and a pressure output end are provided. A control valve 11 is provided between the pressure input end and the pressure controller 4. In this embodiment, the control valve 11 is a three-way valve. The pressure control valve 11 can also be installed in the pressure controller. When the pressure controller 4 is adjusting the pressure, the pressure controller is disconnected from the pressure distribution assembly through the control valve 11. When the pressure reaches a stable state, the corresponding gas path is communicatedthrough the control valve 11.

The function of the pressure distribution plate 1 is to more effectively connect the pressure of the pressure controller 4 to the microfluidic chip to form the gas path. It has a pressure input end and a pressure output end, as well as a passage communicating the pressure input end and the pressure output end. The pressure output end of the pressure distribution plate 1 is connected to the liquid storage pool 34 on the microfluidic chip 3, and further, is connected to the liquid storage pool 34 on the inlet end 32 of the microfluidic chip 3. Usually, a complete flow channel 31 has two inlet ends and a synthesis end 33. The liquid storage pools at the two inlet ends are the liquid storage pools for reagent, and the liquid storage pool at the synthesis end is the liquid storage pool for collecting nanoparticles. The microfluidic chip 3 may be provided with a plurality of independent flow channels 31 to synthesize a plurality of nanoparticles, thereby achieving high throughput.

The pressure controller 4 is a multi-channel pressure controller, the number of the channels is greater than or equal to 2, and the channel refers to the output control pressure channel. The output pressure range of the pressure controller is 0-30 psi or higher, and the accuracy and precision of the pressure value is less than 0.05 psi. For a set of pressure controllers 4, multiple of the microfluidic chip 3 may also be provided in parallel. Generally, the number of pressure output ends on the pressure controller 4 is equivalent to the number of the liquid storage pool for reagent at inlet end on the microfluidic chip 3. The capacity of the liquid storage pool determines the yield of nanosynthesis. In this embodiment, the volume of the liquid storage pool is 20 µl-1 ml.

In order to form an airtight connection between the pressure distribution plate 1 and the microfluidic chip 3, a sealing assembly 2 is provided between the pressure distribution plate 1 and the microfluidic chip 3. The sealing assembly 2 is a sealing gasket, which is provided with an opening and at least covers the top opening of the liquid storage pool to ensure an airtight connection between the pressure distribution plate 1 and the liquid storage pool 34 on the microfluidic chip 3. The sealing gasket may be made of rubber or silica gel material, and the sealing gasket is selectively connected to the pressure distribution plate 1 or to a port of the liquid storage pool 34. When connected to the pressure distribution plate, it is tightly connected to the bottom of the pressure distribution plate 1. Accordingly, a vent hole is provided on the sealing gasket, and the communication is formed between the vent hole and the liquid storage pool and the pressure output end of the pressure distribution plate without hindering the passage of air pressure. Of course, the sealing gasket can also be directly covered on the top opening end of the liquid storage pool 34, and both can achieve an airtight connection between the air pressure distribution plate 1 and the liquid storage pool 34.

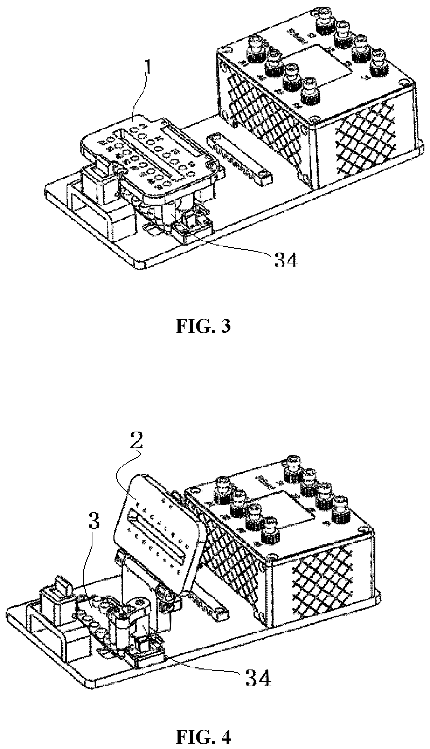

For a better understanding of the present application, one illustrative structure is shown in conjunction with FIG. 3-FIG. 4. When in use, different reagents are loaded into the corresponding liquid storage pool 34, and then the pressure distribution plate 1 is pressed down and closed. The sealing gasket fits tightly against the upper edge of the liquid storage pool 34 to form an airtight contact, and the pressure distribution plate 1 is fixed in a state of airtightly fitting tightly against the liquid storage pool 34 through the lock catch on the mechanism. After the user sets the desired pressure and volume of nanoparticle solution to be produced, the pressure controller is started. When the nanoparticle synthesis reaches the required volume, the program ends automatically. The pressure controller automatically releases the pressure quickly to balance with the atmospheric pressure through the three-way valve, and the locked lock catch is opened to release the pneumatic pressure plate. The user takes out the generated nanoparticle solution and disposes or recycles the chip and liquid storage pool appropriately.

Since the fluid resistance in the pressure distribution assembly is much lower than the flow channel resistance of the microfluidic chip (more than 100 times), the pressure output of the pressure controller 4 is transmitted to the liquid in the liquid storage pool 34 almost instantaneously (<1s), and the flow rate of the fluid in the microfluidic channel and the pressure output are completely one-to-one corresponding. At the same time, the fluid in the microfluidic channel is laminar flow, so the flow rate can be predicted and calculated by simple fluid calculations. Therefore, the present application can achieve the control of the flow rate of the fluid in the flow channel in the microfluidic chip by accurately controlling the pressure, thereby achieving the accurate control of the size of the synthesized nanoparticles. Furthermore, the microfluidic chip described in this application can also be used in patent application number 2021110609590. The advantage of this is that the nanosynthesis formula optimized by the user in this system can be directly used in the low-throughput but high-yield continuous production device of the aforementioned invention application.

In order to better verify the effectiveness of this application, the following nanoparticle synthesis test was performed:
12.5 mM lipid mixture was dissolved in ethanol and loaded into one liquid storage pool 34 as one reagent. The nucleic acid was dissolved in an aqueous buffer solution and loaded into another liquid storage pool 34 as another reagent. By adjusting the pressure output of the pressure controller 4, the flow rate of the water phase solution to that of oil phase solution can reach 4:1, and the total flow rate is 3 mL/min. After adjusted, the pressure output can be stored in the controller as a fixed variable for later use. In order to prevent the reagent in the liquid storage pool from being emptied, it is usually necessary to add excess reagent into the liquid storage pool. For example, if 200 µL of mRNA-encapsulated lipid nanoparticles are synthesized, it is necessary to add 190 µL of mRNA-containing aqueous buffer solution into one liquid storage pool and 70 µL of lipid ethanol solution intoanother liquid storage pool. After the synthesis is completed, 160 µL of aqueous solution and 40 µL of lipid ethanol solution will be consumed, and 30 µL of solution will remain in each liquid storage pool, and finally 200 µL of lipid nanoparticles will be collected.

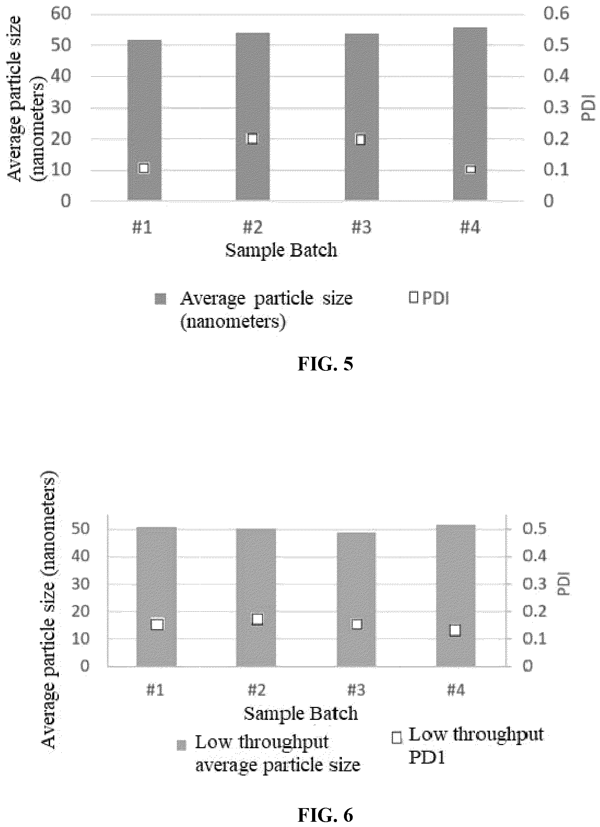

In conjunction with FIG. 5, which shows the results of four independent experiments (all conditions are the same), it can be seen that the nanoparticles synthesized in the four experiments are very similar in size and PDI. This shows that although the system is pressure controlled, the flow rate in the flow channel of the chip can be controlled by precise pressure control output each time, thereby accurately controlling the size, distribution and consistency of the nanoparticles.

We also compared the nanoparticles synthesized using the present system and a previously developed nanoparticle synthesis device (low throughput flow rate control, patent application number 2021110609590). It is worth pointing out that in comparison, due to the compatibility of the device with the microfluidic chip, we can use the same chip for experimental comparison. The previously developed device adopted flow rate control, flow meters and some elements forcontrolling fluid was used, thus the dead volume was relatively large and the minimum synthesis volume was 1 mL. FIG. 6 shows the measurement results of the size and particle distribution PDI of nanoparticles synthesized four times under the same conditions (total flow rate is 3 mL/min, flow rate ratio of water to oil is 4:1) using the previously flow rate controlled device. It can be seen that the size and particle size distribution of the nanoparticles synthesized using the flow rate controlled device are very close to the nanoparticles obtained by the device of the present application (pressure control). The average particle diameter of the nanoparticles synthesized by this system is slightly larger (5%). This difference is probably due to that the actual flow rate and flow rate ratio under pressure control are slightly different from the preset ones, but such a difference is within an acceptable range.

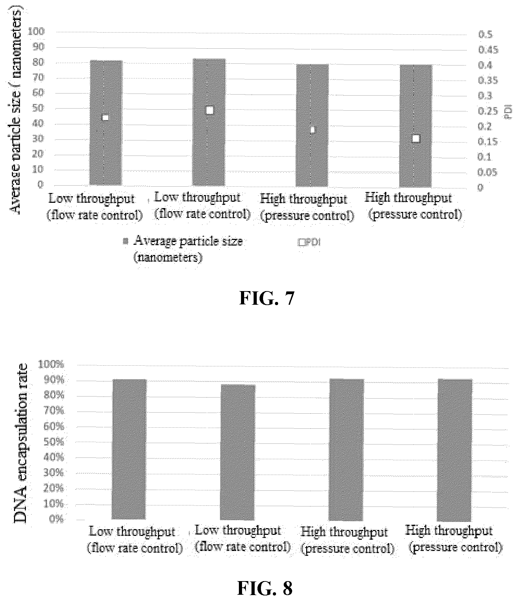

By this system, experimental studies on the simultaneous encapsulation of DNA in the synthesis of lipid nanoparticles was carried out. We measured the size of the lipid nanoparticles encapsulating DNA and the DNA encapsulation rate, and compared them with the lipid nanoparticles encapsulated with DNA synthesized using a low-throughput device with flow rate control. The results in FIG. 7 show that the size and PDI of the lipid nanoparticles encapsulating DNA synthesized under the two devices are relatively close, with an average particle size of about 80 nm and a PDI of about 0.2. Further studies on the encapsulation rate showed that the encapsulation rate of DNA by the lipid nanoparticles synthesized on the two devices reached about 90%, as shown in FIG. 8.

In vitro cell transfection studies were conducted using lipid nanoparticles encapsulating green fluorescent protein (GFP) DNA synthesized by this system in HepG2 (human liver cancer cell line) and K562 (human immortalized myeloid leukemia cell line), respectively. 24 hours before transfection, HepG2 and K562 cells were seeded in 96-well plates at a concentration of 2-4 x 10⁴ cells/well. On the day of transfection, lipid nanoparticles encapsulating GFP DNA plasmid were added to the culture medium of HepG2 or K562 cells at a concentration of 400 ng DNA/well.

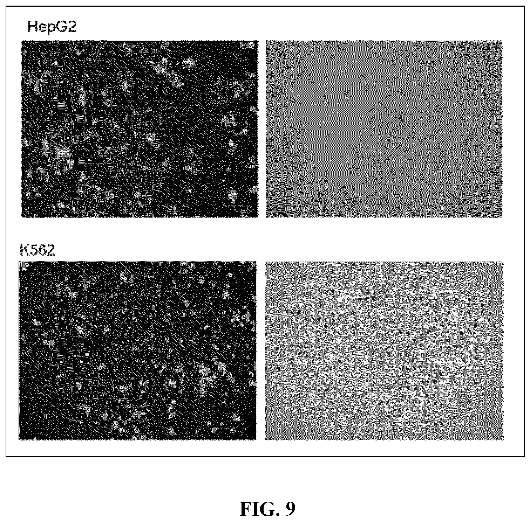

48 hours after transfection, HepG2 cells were treated with Hoechst 33342 (1 µg/ml) for nucleus staining, and green (green fluorescent protein GFP expressed in the cytoplasm) and blue (nucleus) fluorescence images were recorded using a BioRad Zoe fluorescence cell imager. Selected green field/blue field overlay images and bright field images are shown in FIG. 9 (top). Green fluorescence field and bright field images of K562 cells are shown in FIG. (bottom). The experimental results show that the lipid nanoparticles encapsulating DNA synthesized by this system can successfully transfect DNA into cells and express it in vitro.

In summary, this system can realize the small-volume (<0.2mL) and high-throughput (two or more samples can be prepared at the same time) nanoparticle synthesis, thereby saving a large amount of raw materials and reducing the number of experiments for users. It is suitable for the selection and optimization of nanosynthesis formulas. At the same time, the microfluidic chip in this system can be compatible with the previously invented low-throughput and high-yield device based on flow rate control. Users can directly use the optimized formula on the flow rate control device to obtain similar nanoparticles.

Finally, it should be noted that the terms "up", "down", "left", "right", "vertical", "horizontal", "inside", "outside", etc. indicate directions or positional relationships based on the directions or positional relationships shown in the accompanying drawing, only for the convenience of describing the present application and simplifying the description, and do not indicate or imply that the device or element referred to must have a specific direction, be constructed and operated in a specific direction. Therefore, they cannot be understood as limitations on the present application. Furthermore, the terms "first", "second", and "third" are used for descriptive purposes only and should not be understood as indicating or implying relative importance.

The above embodiments are only used to illustrate the technical solutions of the present application, rather than to limit them. Although the present application has been described in detail with reference to the aforementioned embodiments, those ordinary skilled in the art should understand that they can still modify the technical solutions described in the aforementioned embodiments, or make equivalent replacements for some of the technical features therein. However, these modifications or replacements do not deviate the essence of the corresponding technical solutions from the spirit and scope of the technical solutions of the embodiments of the present application.

## Claims

1. A microfluidic chip based on microfluidic technology, comprising a chip body, wherein at least one flow channel for synthesizing nanoparticles is provided on the chip body, wherein each of an inlet end and a synthesis end of the flow channel is provided with a liquid storage pool, and a central axis of the liquid storage pool is perpendicular to the flow channel.

2. The microfluidic chip based on microfluidic technology of claim 1, wherein the liquid storage pool is integrally formed or detachably connected with the chip body.

3. The microfluidic chip based on microfluidic technology of claim 2, wherein the detachable connection is formed between the liquid storage pool and the microfluidic chip through mutually matched Luer tapers.

4. A high-throughput nanoparticle synthesis system based on microfluidic technology, comprising a pressure controller, and a pressure distribution assembly connected to an output end of the pressure controller, wherein a pressure output end of the pressure distribution assembly is connected to the microfluidic chip in any one of claims 1-3, and the number of the microfluidic chip is at least one.

5. The high-throughput nanoparticle synthesis system based on microfluidic technology of claim 4, wherein a sealing assembly is provided between the pressure distribution assembly and the microfluidic chip to form an airtight connection between the pressure distribution assembly and the microfluidic chip.

6. The high-throughput nanoparticle synthesis system based on microfluidic technology of claim 5, wherein the pressure distribution assembly comprises a pressure distribution plate, and the sealing assembly is a sealing gasket, which is provided with an opening and at least covers a top opening of the liquid storage pool to ensure the airtight connection between the pressure distribution assembly and the microfluidic chip.

7. The high-throughput nanoparticle synthesis system based on microfluidic technology of claim 5, wherein the sealing gasket is selectively connected to the pressure distribution plate or to a port of the storage pool.

8. The high-throughput nanoparticle synthesis system based on microfluidic technology of claim 4, wherein a volume of the liquid storage pool is 20_{[}tl-lml.

9. The high-throughput nanoparticle synthesis system based on microfluidic technology of claim 4, wherein the pressure controller is a multi-channel pressure controller, and the number of the channels is greater than or equal to 2.

10. The high-throughput nanoparticle synthesis system based on microfluidic technology of claim 4, wherein a control valve is provided between the pressure controller and the pressure distribution assembly.
